# EUROPÄISCHE PATENTSCHRIFT

(11) **EP 2 164 436 B1**
(45) Veröffentlichungstag und Bekanntmachung des Hinweises auf die Patenterteilung: **25.09.2013**
(21) Anmeldenummer: 08758772.1
(22) Anmeldetag: 27.05.2008
(51) Int. Cl.: A61F 13/00

(54) **LAGERSTABILE LAMINATABSCHNITTE**
STORAGE-STABLE LAMINATE SEGMENTS
PORTIONS DE STRATIFIE STABLE

(30) Priorität: 04.06.2007 DE 102007025973
(43) Veröffentlichungstag der Anmeldung: 24.03.2010
(73) Patentinhaber: LTS LOHMANN Therapie-Systeme AG, 56626 Andernach (DE)
(72) Erfinder: SAHM, Hans-Dieter, 57610 Ingelbach (DE); THÖING, Heinrich, 53474 Bad Neuenahr-Ahrweiler (DE); LAUX, Wolfgang, 65582 Diez (DE)
(74) Vertreter: Schweitzer, Klaus
(86) Internationale Anmeldenummer: PCT/EP2008/004185
(87) Internationale Veröffentlichungsnummer: WO 2008/148477

(56) Entgegenhaltungen:
- EP-A- 0 040 862
- WO-A-02/089717
- WO-A-2004/054638
- US-A- 5 336 162
- US-A- 5 891 463
- US-A1- 2005 037 059

## Beschreibung

Die vorliegende Erfindung betrifft haftklebend ausgerüstete Laminat- oder Substratabschnitte, deren haftklebende Seite mit einer Schutzabdeckung versehen ist. Bevorzugt handelt es sich bei den genannten Laminat- oder Substratabschnitten um Transdermale Therapeutische Systeme (TTS), insbesondere um therapeutische (wirkstoffhaltige) Pflaster.

Die Herstellung und Verwendung von haftklebenden Laminatabschnitten ist bekannt. So ist z.B. in den meisten Fällen bei einem TTS die haftklebend ausgerüstete Seite, d.h. die bei der Anwendung auf der Haut des Patienten haftende Seite, mit einer Schutzfolie, einem sog. Release-Liner, abgedeckt, um ein Verkleben der Abschnitte untereinander oder mit der äußeren Verpackung zu verhindern. Üblicherweise ist zudem die Schutzfolie mit einem meist mittigen Schnitt versehen, der ein leichtes Abziehen der Folie in mindestens zwei Abschnitten ermöglicht und daher als Abziehhilfe dient. Solche Systeme sind z.B. in der DE 3344335 A1 beschrieben.

Der Kleber der haftklebend ausgerüsteten Seite eines solchen Laminatabschnitts oder TTS besitzt jedoch ein von der Viskosität bei Raumtemperatur abhängiges Fließverhalten, das als kalter Fluss bezeichnet wird. Dies kann dazu führen, dass der Kleber bei Lagerung der Abschnitte an der Schnittkante austritt und es so zu einer äußerst nachteiligen Verklebung der Abschnitte untereinander oder mit der Umverpackung, meist einem Packstoffbeutel, kommt.

Eine mögliche Lösung für dieses Problem ist in der DE 19925338 A1 beschrieben. Hier ist die Schutzfolie auf der Haftklebeschicht so ausgebildet, dass sie aus zwei Folienabschnitten besteht, die sich im Bereich der Schnittkante überlappen. Diese Maßnahme verhindert jedoch insbesondere z.B. bei TTS-Produkten mit stark zum Kleberaustritt neigenden Formulierungen nicht in allen Fällen ein Verkleben von TTS-Produkt mit der Verpackung. Weiterhin erweist sich die Herstellung solcher Produkte als verbesserungswürdig. Bei deren Herstellung wird das TTS-Laminat, bestehend aus Rückschicht, Matrix und Zwischenabdeckung durch die Rückschicht und Matrix konturgestanzt und nach Entfernung des überstehenden Restlaminates werden die gestanzten TTS einzeln mit Hilfe des Zwischenträgers / -abdeckung auf eine neue Schutzfolie (Release-Liner) übertragen. Diese Schutzfolie weist zur Verhinderung eines Kleberaustritts im Bereich der Schnittkante eine Überlappung auf. Dieses Verfahren ist störanfällig, da zahlreiche Materialien gleichzeitig bewegt und exakt positioniert werden müssen. Nachteilig ist weiterhin, dass der Zwischenträger entfernt und entsorgt werden muss.

Die US-A 2005 037059 beschreibt ein haftklebend ausgerüstetes Mehrschichtensystem für die Applikation von Wirkstoffen auf die Haut oder Schleimhaut, das sich in der Form einer Doppelscheibe verkörpert. Eine äußere Schicht fungiert als primäre Klebeschicht zum Aufkleben des Pflasters auf den Probanten. Auf der Kleberseite der äußeren Schicht ist eine Trennschicht angeordnet, die aus zwei einzelnen Teile besteht, die durch einen Spalt ("slit") getrennt sind und die eine zentrale Öffnung bilden. Die Trennschicht hemmt die Wirkstoffmigration aus der Wirkstoff enthaltenden, inneren Schicht in die äußere Klebeschicht.

Die US-A 5 336 162 betrifft eine medizinische Bandage, in der ein Schlitz oder ein auf Stoß ("butting") angeordnetes, zweiteiliges Trägermaterial in der dortigen Zeichnung in Erscheinung tritt. Dieses Trägermaterial steht nicht in Kontakt mit der druckempfindlichen Kleberschicht und der Rückschicht, sondern es befindet sich noch eine als folienförmiges Substart bezeichnete Lage dazwischen.

Aus der EP-A 0 040 862 ist eine Brandwundenmatrix bestehend aus mehreren Schichten bekannt. Sie enthält u.a. eine Deckschicht und eine Rückschicht ("backing member"), wobei eine Diffusionsmatrix mit einem darin verteilten Arzneimittel in einer Aushöhlung in der Rückschicht angeordnet ist.

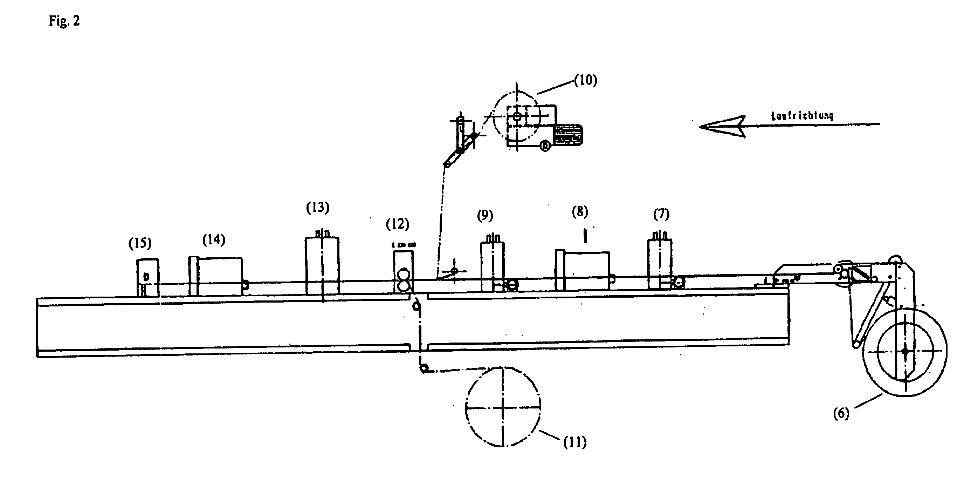

Die US-A 5 891 463 betrifft ein System zur Applikation von Arzneimitteln, das einen offenporigen Schaum und eine für Dampf durchlässige, für Flüssigkeiten aber undurchlässige Membran enthält. Die dortige Figur 2 illustriert eine im Randbereich angeordnete Klebeschicht und eine Trennschicht mit einem großen Fenster, mit der die Klebeschicht abgedeckt ist.

Die WO 2004/054638 beschreibt eine Inhaltsstoff-Abgabevorrichtung, das ein Trägerelement, welches ein Inhaltsstoff Aufnahmereservoir bildet, und eine Abdeckung für das Reservoir aufweist, wobei Reservoir und Abdeckung aus einem Material hergestellt sind, das im Wesentlichen undurchlässig für im Reservoir enthaltene Inhaltsstoffe ist, wobei die Abdeckung (eine) Öffnung(en) aufweist, so dass von dem Reservoir abzugebende Inhaltsstoffe durch die Öffnung(en) strömen können.

Die WO 02/089717 beschreibt einen Applikator für transdermale Pflaster. Der Applikator weist einen starren Release-Liner und ein lösbar damit verbundenes flexibles transdermales Pflaster auf. Der Release-Liner hat einen ersten und zweiten Abschnitt, die durch einen Schlitz getrennt sind. An den Abschnitten sind Laschen als Ziehhilfen zum Entfernen der Schutzfolie angebracht.

Die WO 2007/067363 beschreibt eine Zusammensetzung zum haftklebenden Anbringen auf der Haut, die eine Trägerschicht, eine Kleberschicht und eine abziehbare Schutzschicht umfasst, wobei die abziehbare Schutzschicht mit einer Öffnung versehen ist. Ferner umfasst diese Zusammensetzung noch einen inneren Teil, der eine flexible, wirkstoffhaltige Schicht und eine wirkstoffundurchlässige Schicht enthält. Die Öffnung in der abziehbaren Schutzschicht soll dazu dienen, einen Teil der Klebstoffschicht freizulegen und so den Zusammenhalt des inneren Teils mit dem äußeren Teil durch Verkleben zu gewährleisten.

Aufgabe der vorliegenden Erfindung war daher die Bereitstellung von lagerstabilen, haftklebend ausgerüsteten Laminatabschnitten, insbesondere von lagerstabilen TTS-Produkten, bei denen bzw. bei deren Herstellung die aus dem Stand der Technik bekannten Nachteile vermieden werden.

Gelöst wird diese Aufgabe durch die erfindungsgemäßen haftklebend ausgerüsteten Laminatabschnitte (1) umfassend eine Matrix (2) mit einer Rückschicht, eine auf der haftklebenden Seite der Matrix (2) befindliche Schutzfolie (3), die einen Schnitt aufweist, und eine auf der Schutzfolie (3) aufgebrachte, teilweise mit einem Kleber (4) beschicfitete Abdeckfolie (5), die den Schnitt in der Schutzfolie (3) mit dem kleberfreien Teil der Abdeckfolie (5) ( = Abdeckstreifen) überdeckt.

Dieser Abdeckstreifen ist bevorzugt so ausgebildet, dass er den Schnitt in der Schutzfolie vollständig überdeckt und einseitig mit Kleber beschichtet ist, damit er auf der Schutzfolie haftet. Der Abdeckstreifen ist so angebracht, dass er die Funktion des Schnitts in der eigentlichen Schutzfolie als Abziehhilfe nicht beeinträchtigt. D.h. der Abdeckstreifen ist nicht vollständig mit Kleber beschichtet, und der mit Kleber beschichtete Teil befindet sich nur auf einer Seite des Schnitts. Bevorzugt ist daher die Abdeckfolie nur etwa über die Hälfte ihrer Breite mit Kleber versehen. Grundsätzlich kann für den Abdeckstreifen jeder geeignete Kleber verwendet werden, wie z.B. Haftkleber auf Basis von Natur- und Synthese-Kautschuken, Polyacrylaten, Polyester, Polychloroprenen, Polyisobutenen, Polyvinylether und Polyurethanen, die in Kombination mit Zusätzen wie Harzen, Weichmachern und/oder Antioxidantien eingesetzt werden. Bevorzugt wird ein Silikonkleber verwendet. Dies gilt insbesondere für erfindungsgemäße TTS-Produkte, da die meisten Pharmawirkstoffe nur eine sehr geringe Löslichkeit in diesen Silikonklebern aufweisen. Weiterhin bevorzugt ist, dass die Rückseite des Abdeckstreifens mit einer Sperrschicht beschichtet ist, die nicht mit dem Kleber des Abdeckstreifens verkleben kann. Dies gilt insbesondere für TTS-Produkte mit Silikonkleber beschichteten Abdeckstreifen. Geeignete Materialien für solche Sperrschichten sind z.B. Polyethylenterephthalate und Polyethylene.

In einer bevorzugten Ausführungsform entspricht die Größe der Abdeckfolie der Größe der eigentlichen Schutzfolie, die wiederum größer sein kann als der eigentliche Laminatabschnitt oder die Matrix. In diesem Fall überragen Schutzfolie und Abdeckfolie den eigentlichen Laminatabschnitt an dessen Rändern; dies kann als zusätzliche Abziehhilfe für die Schutzfolie dienen und entspricht ebenfalls einer bevorzugten Ausgestaltung der vorliegenden Erfindung.

Der Begriff Schnitt in der eigentlichen Schutzfolie ist weit auszulegen, d.h. er ist nicht z.B. auf einen geraden Schnitt pro Laminatabschnitt beschränkt. Vielmehr sind hier alle geometrischen Formen und Muster (z.B. mehrere Schnitte) umfasst. Beispiele hierfür sind in der DE 3344335 A1 offenbart. Die Abdeckfolie bzw. die Bereiche ihrer Kleberbeschichtung sind dann entsprechend anzupassen.

Die vorliegende Erfindung umfasst somit haftklebend ausgerüstete Laminatabschnitte jeder Art (wie z.B. Heftpflaster, Haftklebeetiketten, selbstkelbende Briefmarken etc.) bevorzugt in der Ausprägung eines TTS. Diese Abschnitte bestehen in der Regel aus mehreren Schichten und umfassen eine Matrix (mit oder ohne Rückschicht) mit einer haftklebend ausgerüsteten Seite oder Schicht, daran angrenzend eine Schutzfolie mit Schnitt und darauf eine kleberbeschichtete Abdeckfolie, die den Schnitt bevorzugt vollständig überdeckt. In der Ausprägung als TTS umfasst ein solcher Laminatabschnitt in der Regel eine Rückschicht, eine wirkstoffhaltige haftklebend ausgerüstete Matrix (evtl. mit separater Haftklebeschicht) zum Aufbringen auf der Haut des Patienten, eine mit Schnitt versehene Schutzfolie und eine Abdeckfolie mit Kleberschicht. Bei den genannten Laminatabschnitten können weitere Schichten hinzutreten bzw. die genannten Elemente können verschiedene Schichten umfassen. Die vorliegende Erfindung ist breit einsetzbar und insbesondere bei allen haftklebend ausgerüsteten TTS verwendbar.

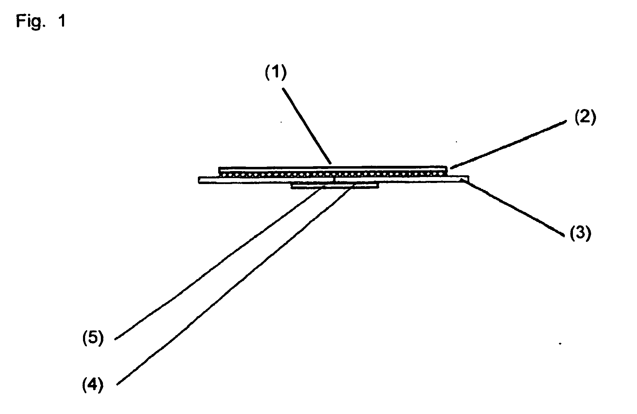

Fig. 1 zeigt ein Beispiel für den Aufbau eines erfindungsgemäßen Laminatabschnitts (1). Es bedeuten: (2) Matrix, (3) Schutzfolie (Release-Liner), (4) Kleberschicht auf Abdeckfolie und (5) Abdeckfolie.

Die vorliegende Erfindung umfasst auch ein Verfahren zur Herstellung der erfindungsgemäßen Laminatabschnitte (1) bzw. TTS-Produkte (TTS) umfassend die Schritte:
a) Versehen der Schutzfolie (3) eines haftklebend ausgerüsteten Laminats mit einem Schnitt,
b) Konturstanzen der Laminatabschnitte (1) ohne die Schutzfolie (3) zu durchtrennen,
c) Entfernen des erstehenden Materials zwischen den Laminatabschnitten (1),
d) Aufbringen der mit Kleber vorbeschichteten Abdeckfolie (5) auf die einen Schnitt aufweisende Schutzfolie (3) und
e) Ausschneiden der Laminatabschnitte (1).

Am Beispiel eines TTS bestehend aus Rückschicht, Matrix und Schutzfolie wird das erfindungsgemäße Verfahren näher erläutert ohne auf diese Ausführungsform beschränkt zu sein. Im Gegensatz zum beschriebenen Stand der Technik dient die Schutzfolie des Ausgangslaminats nun nicht mehr nur als Zwischenträger, der später entsorgt werden muss, sondern ist bereits Bestandteil des Endprodukts. Das TTS-Laminat wird von einer Rolle gezogen und zunächst von seiner Unterseite (Schutzfolienseite) so angestanzt, dass die Schutzfolie in Längsrichtung mit einem linearen Schnitt so versehen wird, dass Matrix und Rückschicht unverletzt bleiben. In einem kontinuierlich erfolgenden weiteren Schritt wird eine Konturstanzung durch die Rückschicht und die Matrix vorgenommen, wobei die Schutzfolie unverletzt bleibt und anschließend das überstehende Material aus Rückschicht und Matrix entfernt (abgegittert) werden kann. Danach wird die mit Kontaktkleber vorbeschichtete Abdeckfolie als schmaler Streifen auf die Unterseite des TTS, auf die Schutzfolie, aufgebracht. Bevorzugt überdeckt die Abdeckfolie dabei die Schutzfolie des TTS vollständig. Nach einem sich anschließenden Querschnitt zum Ausschneiden der einzelnen Laminatabschnitte können die TTS-Abschnitte direkt in die äußere Verpackung, meist in Form eines Siegelbeutels, abgefüllt werden. Im Gegensatz zu dem beschriebenen Herstellungsverfahren von Laminatabschnitten mit sich überlappender Schutzfolie ist bei dem erfindungsgemäßen Verfahren die Zufuhr weiterer Materialien nicht mehr erforderlich.

Wie ausgeführt, ist die Abdeckfolie nicht vollständig mit Kleber beschichtet, um die Funktion des Schnitts in der Schutzfolie als Abziehhilfe nicht negativ zu beeinträchtigen. Die bevorzugten Verfahren für die Herstellung der Abdeckfolie sind demzufolge Rollenbeschichtungsverfahren mit ausgesparten Zonen aus denen sich durch einfachen Rollenschnitt die benötigten Abdeckstreifen herstellen lassen. Als Material für die Abdeckfolie ist grundsätzlich eine Vielzahl von Materialien geeignet, wie z.B. silikonisiertes Polyethylenterephthalat, Polyethylenterephthalat, Polyethylen, Polypropylen. Bevorzugt entspricht das Material der Abdeckfolie dem Material, das für die eigentliche Schutzfolie verwendet wird.

Fig. 2 zeigt eine mögliche Ausgestaltung des erfindungsgemäßen Herstellungsverfahrens. Dabei bedeuten: (6) Laminatrolle, (7) Stanze für Anstanzung der Schutzfolie zur Erzeugung des Schnitts, (8) Vorzug 1, (9) Konturstanze, (10) Aufwickler für Stanzgitter, (11) Abrollung für Abeckstreifen bzw. Abdeckfolie, (12) Kaschierstation, 13 (Prägestanze), (14) Vorzug 2 und (15) Querschneider.

Der Vorteil des erfindungsgemäßen Herstellverfahrens besteht insbesondere in der Ersparnis des neu zuzuführenden endgültigen Schutzfolienstücks (Vermeidung eines Zwischenträgers) sowie in der leichteren Prozessführung und damit in einer schnelleren und sichereren Handhabung des Produkts, Die Ablösung der eigentlichen Schutzfolie wird durch den eingesetzten (auf einer Seite des Schnitts verklebten) Abdeckstreifen sogar noch erleichtert.

Somit kann dieses Verfahren bzw. der erfindungsgemäße Produktaufbau insbesondere bei allen haftklebend ausgerüsteten Produkten zum Einsatz kommen, bei denen auf Grund von stark fließenden Klebermassen ein Austritt des Klebers durch den Schutzfolienschnitt und damit ein unerwünschtes Verkleben von Produkten untereinander oder mit deren Verpackung zu befürchten ist.

## Patentansprüche

1. Haftklebend ausgerüsteter Laminatabschnitt (1) umfassend eine Matrix (2) mit einer Rückschicht, eine auf der haftklebenden Seite der Matrix (2) befindliche Schutzfolie (3), die einen Schnitt aufweist, und eine auf der Schutzfolie (3) aufgebrachte, teilweise mit einem Kleber (4) beschichtete Abdeckfolie (5), die den Schnitt in der Schutzfolie (3) mit dem kleberfreien Teil der Abdeckfolie (5) überdeckt.

2. Laminatabschnitt (1) nach Anspruch 1, **dadurch gekennzeichnet, dass** der Schnitt in der Schutzfolie (3) als Abziehhilfe für die Schutzfolie (3) dient und diese in einen oder mehrere gleich oder verschieden große Abschnitte unterteilt.

3. Laminatabschnitt (1) nach Anspruch 1 oder2, **dadurch gekennzeichnet, dass** die Abdeckfolie (5) den Schnitt in der Schutzfolie (3) mit dem kleberfreien Teil der Abdeckfolie (5) vollständig überdeckt.

4. Laminatabschnitt (1) nach einem oder mehreren der Ansprüche 1 bis 3, **dadurch gekennzeichnet, dass** die Abdeckfolie (5) die Schutzfolie (3) vollständig überdeckt.

5. Laminatabschnitt (1) nach einem oder mehreren der Ansprüche 1 bis 5, **dadurch gekennzeichnet, dass** der Laminatabschnitt (1) als Transdermales Therapeutisches System (TTS) ausgebildet ist.

6. TTS nach Anspruch 5 umfassend eine Rückschicht, eine Matrix (2), eine Schutzfolie (3) mit Schnitt auf der haftklebenden Seite der Matrix (2) und eine auf der Schutzfolie (3) aufgebrachte Abdeckfolie (5), die den Schnitt in der Schutzfolie (3) überdeckt.

7. TTS nach Anspruch 5 oder 6, **dadurch gekennzeichnet, dass** die Abdeckfolie (5) mit einem Silikonkleber beschichtet ist.

8. TTS nach einem oder mehreren der Ansprüche 5 bis 7, **dadurch gekennzeichnet, dass** die Rückseite der Abdeckfolie (5) mit einer Sperrschicht versehen ist, die nicht mit dem Kleber der Abdeckfolie (5) verklebt.

9. Verfahren zur Herstellung eines Laminatabschnitts (1) oder TTS nach einem oder mehreren der Ansprüche 1 bis 8 umfassend die Schritte
a) Versehen der Schutzfolie (3) eines haftklebend ausgerüsteten Laminats mit einem Schnitt,
b) Konturstanzen der Laminatabschnitte (1) ohne die Schutzfolie (3) zu durchtrennen,
c) Entfernen des erstehenden Materials zwischen den Laminatabschnitten (1),
d) Aufbringen der mit Kleber vorbeschichteten Abdeckfolie (5) auf die einen Schnitt aufweisende Schutzfolie (3) und
e) Ausschneiden der Laminatabschnitte (1).

## Claims

1. Pressure-sensitively adhesively furnished laminate section (1) comprising a matrix (2), with a backing layer; a protective sheet (3) which is located on the pressure-sensitively adhesive side of the matrix (2) and has a slit; and a cover sheet (5) which is applied to the protective sheet (3), which is partly coated with an adhesive (4), and which covers the slit in the protective sheet (3) with the adhesive-free part of the cover sheet (5).

2. Laminate section (1) according to Claim 1, **characterized in that** the slit in the protective sheet (3) serves as a peeling aid for the protective sheet (3) and divides it into one or more segments of the same or different size.

3. Laminate section (1) according to Claim 1 or 2, **characterized in that** the cover sheet (5) fully covers the slit in the protective sheet (3) with the adhesive-free part of the cover sheet (5).

4. Laminate section (1) according to one or more of Claims 1 to 3, **characterized in that** the cover sheet (5) fully covers the protective sheet (3).

5. Laminate section (1) according to one or more of Claims 1 to 5, **characterized in that** the laminate section (1) is constructed as a transdermal therapeutic system (TTS).

6. TTS according to Claim 5, comprising a backing layer, a matrix (2), a protective sheet (3) with a slit on the pressure-sensitively adhesive side of the matrix (2), and a cover sheet (5) which is applied to the protective sheet (3) and which covers the slit in the protective sheet (3).

7. TTS according to Claim 5 or 6, **characterized in that** the cover sheet (5) is coated with a silicone adhesive.

8. TTS according to one or more of Claims 5 to 7, **characterized in that** the back of the cover sheet (5) is provided with a barrier layer which does not stick to the adhesive of the cover sheet (5).

9. Method of producing a laminate section (1) or TTS according to one or more of Claims 1 to 8, comprising the steps of
a) providing the protective sheet (3) of a pressure-sensitivity adhesively furnished laminate with a slit,
b) contour-cutting the laminate sections (1) without severing the protective sheet (3),
c) removing the remaining material between the laminate sections (1),
d) applying the cover sheet (5), precoated with adhesive, to the protective sheet (3) having a slit, and
e) cutting out the laminate sections (1).

## Revendications

1. Portion de stratifié autoadhésif (1) comprenant une matrice (2) avec une couche dorsale, un film de protection (3) qui présente une entaille, se trouvant sur la face autoadhésive de la matrice (2), et un film de recouvrement (5) appliqué sur le film de protection (3) et partiellement revêtu d'une colle (4), qui recouvre l'entaille dans le film de protection (3) avec la partie sans colle du film de recouvrement (5).

2. Portion de stratifié (1) selon la revendication 1, **caractérisée en ce que** l'entaille dans le film de protection (3) sert d'aide à l'enlèvement du film de protection (3) et divise celui-ci en une ou plusieurs parties de grandeurs égales ou différentes.

3. Portion de stratifié (1) selon la revendication 1 ou 2, **caractérisée en ce que** le film de recouvrement (5) recouvre entièrement l'entaille dans le film de protection (3) avec la partie sans colle du film de recouvrement (5).

4. Portion de stratifié (1) selon une ou plusieurs des revendications 1 à 3, **caractérisée en ce que** le film de recouvrement (5) recouvre entièrement le film de protection (3).

5. Portion de stratifié (1) selon une ou plusieurs des revendications 1 à 5, **caractérisée en ce que** la portion de stratifié (1) constitue un Système Thérapeutique Transdermique (STT).

6. STT selon la revendication 5, comprenant une couche dorsale, une matrice (2), un film de protection (3) avec une entaille sur la face autoadhésive de la matrice (2) et un film de recouvrement (5) appliqué sur le film de protection (3), qui recouvre l'entaille dans le film de protection (3).

7. STT selon la revendication 5 ou 6, **caractérisé en ce que** le film de recouvrement (5) est revêtu d'une colle au silicone.

8. STT selon une ou plusieurs des revendications 5 à 7, **caractérisé en ce que** la face arrière du film de recouvrement (5) est munie d'une couche d'arrêt, qui ne colle pas avec la colle du film de recouvrement (5).

9. Procédé de fabrication d'une portion de stratifié (1) ou d'un STT selon une ou plusieurs des revendications 1 à 8, comprenant les étapes suivantes:
a) pratiquer une entaille dans le film de protection (3) d'un stratifié autoadhésif,
b) marquer le contour des portions de stratifié (1) sans trancher le film de protection (3),
c) enlever le matériau qui apparaît entre les portions de stratifié (1),
d) appliquer le film de recouvrement (5), préalablement revêtu de colle, sur le film de protection (3) présentant une entaille, et
e) découper les portions de stratifié (1).
